# EUROPEAN PATENT SPECIFICATION

(11) **EP 4 543 534 B1**
(45) Date of publication and mention of the grant of the patent: **17.06.2026**
(21) Application number: 23733567.4
(22) Date of filing: 23.05.2023
(51) Int. Cl.: A61N 1/05, A61N 1/36

(54) **SYSTEMS FOR CONTROLLING AND OPTIMIZING CLOSED-LOOP NEUROMODULATION THERAPY**
SYSTEME ZUR STEUERUNG UND OPTIMIERUNG EINER GESCHLOSSENEN NEUROMODULATIONSTHERAPIE
SYSTÈMES DE COMMANDE ET D'OPTIMISATION D'UNE THÉRAPIE DE NEUROMODULATION EN BOUCLE FERMÉE

(30) Priority: 22.06.2022 US 202263354567 P; 22.05.2023 US 202318200269
(43) Date of publication of application: 30.04.2025
(73) Proprietor: Medtronic, Inc., Minneapolis, MN 55432-5604 (US)
(72) Inventor: CLELAND, Andrew Jay, Minneapolis, Minnesota 55432-5604 (US); ZENISEK, Todd D., Minneapolis, Minnesota 55432-5604 (US); SCHMELING, Andrew L., Minneapolis, Minnesota 55432-5604 (US); HINCAPIE, Juan G., Minneapolis, Minnesota 55432-5604 (US); LITVAK, Leonid M., Minneapolis, Minnesota 55432-5604 (US)
(74) Representative: Zimmermann & Partner Patentanwälte mbB
(86) International application number: PCT/US2023/067336
(87) International publication number: WO 2023/250241

(56) References cited:
- US-A1- 2016 121 124
- US-A1- 2019 366 094
- US-A1- 2021 339 014
- US-A1- 2022 134 109

## Description

### BACKGROUND

The present disclosure is directed to therapeutic neuromodulation and relates more particularly to controlling and optimizing closed-loop neuromodulation therapy.

Closed-loop neuromodulation therapy may be carried out by sending an electrical signal generated by a pulse generator to a stimulation target (e.g., nerves, non-neuronal cells, etc.), which may provide a stimulating or blocking therapy to the stimulation target. In such closed-loop neuromodulation therapies, one or more signals resulting from the stimulating or blocking therapy may be recorded and the therapy may be adjusted based on the recorded signals. Document US 2016/121124 A1 relates to stimulation devices.

### BRIEF SUMMARY

The invention is defined by the appended claims. Example aspects of the present disclosure include:
A system for controlling a therapeutic procedure according to at least one embodiment of the present disclosure comprises a pulse generator configured to generate an electrical signal; a lead in communication with the pulse generator and configured to transmit the electrical signal to a plurality of electrodes; and the plurality of electrodes in communication with the lead and configured to be implanted near a stimulation target, the plurality of electrodes comprising at least one stimulating electrode and at least one recording electrode, the at least one stimulating electrode configured to stimulate an anatomical element based on the electrical signal and the at least one recording electrode configured to record a physiological response, wherein the at least one pair of recording electrodes is substantially perpendicular to the at least one pair of stimulating electrodes.

Any of the aspects herein, wherein the at least one recording electrode is proximal to the at least one stimulating electrode.

Any of the aspects herein, wherein the at least one recording electrode is distal to the at least one stimulating electrode.

Any of the aspects herein, wherein a first recording electrode of the at least one stimulating electrode is proximal to the at least one stimulating electrode and a second recording electrode of the at least one stimulating electrode is distal to the at least one stimulating electrode.

Any of the aspects herein, wherein the lead comprises a directional lead and wherein the at least one stimulating electrode directs the stimulation in a target direction.

Any of the aspects herein, wherein the lead comprises a directional lead and wherein the at least one recording electrode records the physiological response in a target direction.

Any of the aspects herein, wherein the at least one stimulating electrode comprises a pair of stimulating electrodes, each stimulating electrode being spaced apart from one another at a first distance and the at least one pair of stimulating electrodes are positioned at a second distance from the at least one recording electrode.

Any of the aspects herein, wherein the lead comprises a cylindrical lead having segments and the at least one recording electrode comprises at least a half segment.

Any of the aspects herein, wherein the at least one recording electrode has a surface area larger than the at least one stimulating electrode.

Any of the aspects herein, further comprising: a processor; and a memory storing data for processing by the processor, the data, when processed, causes the processor to: generate an electrical signal using the pulse generator; stimulate the anatomical element based on the electrical signal; record the physiological response from the stimulation; and
adjust the electrical signal based on the recorded physiological response.

A system for controlling a therapeutic procedure according to at least one embodiment of the present disclosure comprises a pulse generator configured to generate an electrical signal; a first lead in communication with the pulse generator and configured to transmit the electrical signal to at least one electrode; at least one stimulating electrode positioned on the first lead and configured to stimulate an anatomical element based on the electrical signal, the at least one stimulating electrode to be implanted at a first positioned near the anatomical element; a second lead; and at least one recording electrode positioned on the second lead and configured to record a physiological response to the stimulation, the at least one recording electrode to be implanted at a second positioned near the at least one stimulating electrode.

Any of the aspects herein, wherein the at least one recording electrode is at least one of implanted epidurally or implanted intrathecally.

Any of the aspects herein, wherein the at least one recording electrode is disposed on a pump catheter.

Any of the aspects herein, wherein the at least one stimulating electrode comprises a pair of stimulating electrodes, each stimulating electrode of the at least one stimulating electrode is spaced apart from each other at a first distance and the at least one pair of stimulating electrodes are positioned at a second distance from the at least one recording electrode.

Any of the aspects herein, wherein the at least one recording electrode is perpendicular to the at least one stimulating electrode.

Any of the aspects herein, wherein the first lead comprises a directional lead wherein the at least one stimulating electrode direct the stimulation in a target direction.

Any of the aspects herein, wherein the second lead comprises a directional lead wherein the at least one recording electrode records the physiological response in a target direction.

Any of the aspects herein, wherein the at least one recording electrode has a surface area larger than the at least one stimulating electrode.

Any of the aspects herein, further comprising: a processor; and a memory storing data for processing by the processor, the data, when processed, causes the processor to: generate an electrical signal using the pulse generator; stimulate the anatomical element based on the electrical signal; record the physiological response from the stimulation; and
adjust the electrical signal based on the recorded physiological response.

A lead for stimulating a target according to at least one embodiment of the present disclosure comprises a plurality of electrodes positioned on the lead and configured to be implanted near an anatomical element, the plurality of electrodes comprising at least one stimulating electrode and at least one recording electrode, the at least one stimulating electrode configured to stimulate a stimulating target and the at least one recording electrode configured to record a physiological response resulting from the stimulation, wherein the at least one recording electrode is perpendicular to the at least one stimulating electrode, wherein a first recording electrode of the at least one stimulating electrode is proximal to the at least one stimulating electrode and a second recording electrode of the at least one stimulating electrode is distal to the at least one stimulating electrode, and wherein the at least one stimulating electrode comprises a pair of stimulating electrodes, each stimulating electrodes of the at least one pair of stimulating electrodes is spaced apart from each other at a first distance and the at least one pair of stimulating electrodes are positioned at a second distance from the first recording electrode and at a third distance from the second recording electrode.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a diagram of a system according to at least one embodiment of the present disclosure;
Fig. 2 is a diagram of an additional system according to at least one embodiment of the present disclosure;
Fig. 3A depicts a schematic illustration of a lead according to at least one embodiment of the present disclosure;
Fig. 3B depicts a schematic illustration of a lead according to at least one embodiment of the present disclosure;
Fig. 3C depicts a schematic illustration of a cross-sectional view of a lead according to at least one embodiment of the present disclosure;
Fig. 3D depicts a schematic illustration of a cross-sectional view of a lead according to at least one embodiment of the present disclosure;
Fig. 3E depicts a schematic illustration of a cross-sectional view of a lead according to at least one embodiment of the present disclosure;
Fig. 3F depicts a schematic illustration of a cross-sectional view of a lead according to at least one embodiment of the present disclosure;
Fig. 4A depicts a schematic illustration of a lead having a plurality of electrodes in a first configuration according to at least one embodiment of the present disclosure;
Fig. 4B depicts a schematic illustration of a lead having a plurality of electrodes in a second configuration according to at least one embodiment of the present disclosure;
Fig. 4C depicts a schematic illustration of a lead having a plurality of electrodes in a third configuration according to at least one embodiment of the present disclosure;
Fig. 4D depicts a schematic illustration of a lead having a plurality of electrodes in a fourth configuration according to at least one embodiment of the present disclosure;
Fig. 5A depicts a schematic illustration of a lead according to at least one embodiment of the present disclosure;
Fig. 5B depicts a schematic illustration of a lead according to at least one embodiment of the present disclosure;
Fig. 5C depicts a schematic illustration of a lead according to at least one embodiment of the present disclosure;
Fig. 6 is a block diagram of a system according to at least one embodiment of the present disclosure; and
Fig. 7 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., random-access memory (RAM), read-only memory (ROM), electrically erasable programmable ROM (EEPROM), flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

As previously described, in closed-loop neuromodulation therapies, an electrical signal generated by a pulse generator may be sent to a stimulation target. A resulting signal from the electrical signal may be recorded. The resulting signal may provide information by which to adjust the electrical signal. For example, spinal cord stimulation (SCS) is a form of closed-loop neuromodulation in which contacts are placed near the patient's spinal cord and one or more nerves are stimulated to block pain signal(s) sent to the brain from the one or more nerves. During SCS, the patient may move, and thus the spinal cord may also move further away from or closer to the contacts and thus affect the stimulation. To mitigate such undesirable effects, some contacts may be used to stimulate the nerve and other contacts may be used to record an Evoked Compound Action Potential (ECAP) resulting from the stimulation. The ECAP recording can be used to adjust the stimulation in real-time to compensate for movement of the spinal cord to and from the contacts. In other embodiments it may be desirable to record the ECAP whether with movement or without movement as the ECAP may provide information about a pathophysiology of a patient. In such embodiments, the ECAP may also be used to modulate therapy provided based on the ECAP signal. In other words, the ECAP may be used to modulate therapy whether or not there is movement of the patient, contacts, anatomical elements, stimulation target, etc. In other closed-loop neuromodulation therapies, other signals may be recorded. For example, in deep brain stimulation, local field potentials (LFPs) may be recorded in the brain. In various types of therapies provided, the desired signal to be recorded (e.g., ECAPs, LFPs, etc.) may be recorded at a distance from the stimulation, near the stimulation, or from multiple places. However, the recording may include artifacts, which may mask, obscure, or otherwise corrupt at least a portion of the recording and thus, may interfere with the adjusted therapy provided.

Measuring or recording the signal (e.g., ECAPs, LFPs, etc.) involves stimulating a target stimulation and then recording a response to the stimulation from a distance away. The optimal distance to record from is related to a conduction velocity of the target stimulation (which may be, for example, a neuron) and the timing and size of a stimulation artifact that may occur. In conventional lead configurations, stimulation contacts (e.g., electrodes) close to the recording contacts cannot be used because the stimulation artifact obscures the signal to be recorded. In at least one embodiment of the present disclosure, recording with contacts or electrodes in a perpendicular configuration to the stimulation contacts may greatly reduce the stimulation artifact. Recording with contacts or electrodes a distance away from the stimulation contacts may also reduce the stimulation artifact. Such characteristics can be used in the design of a lead by having a number of stimulating contacts or electrodes with spacing and size optimized for therapy delivery and at least one recording contact or electrode spaced a distance away from the stimulation contacts and perpendicular to the stimulating contacts or electrodes to be optimized for signal recording. The at least one recording contact or electrode can be proximal or distal to the stimulating contacts or electrodes, or both proximal and distal to the stimulating contacts or electrodes (proximal would maintain the current implant technique, while distal would require advancing the lead past the stimulation target). It is also possible that the recording contacts or electrodes can be monopolar. In such instances, a pulse generator may act as a return for the signal.

In some embodiments, the at least one recording contact or electrode may be on a cylindrical lead. In such embodiments, the at least one recording contact or electrode may comprise two recording contacts or electrodes and the two recording contacts or electrodes may make up each half of a segmented contact. In other embodiments, the at least one recording contact or electrode may comprise a single directional contact, one or more directional 1/3 circumference contacts, and/or one or more directional 1/4 circumference contacts. In embodiments where the at least one recording contact or electrode comprise two or more contacts, the contacts may be spaced apart an axial inter-contact distance. Additionally, the stimulation contacts or electrodes can be directional (e.g., segmented) for improved stimulation and/or ECAP generation.

In alternative embodiments, the at least one recording contact or electrode can be placed on a separate recording lead with a limited number of contacts or electrodes that can be implanted epidurally or intrathecally. If implanted intrathecally, the at least one recording contact or electrode can be on a pump catheter.

Embodiments of the present disclosure provide technical solutions to one or more of the problems of (1) treating chronic pain using spinal cord stimulation, (2) improving lead designs to reduce or eliminate stimulation artifacts when recording physiological response(s) from stimulating one or more nerves, (3) improving patient outcomes.

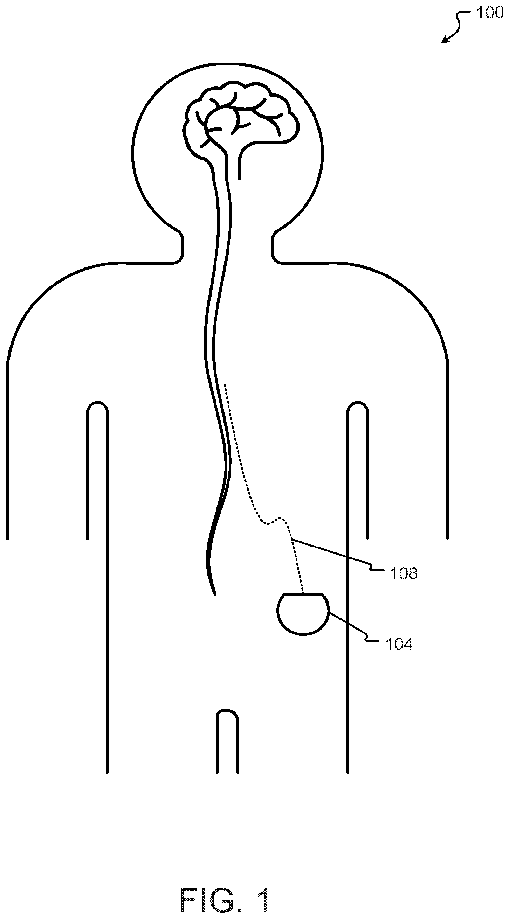

Turning to Fig. 1, a diagram of aspects of a system 100 according to at least one embodiment of the present disclosure is shown. The system 100 may be used to provide electric signals for a patient and/or carry out one or more other aspects of one or more of the methods disclosed herein. For example, the system 100 may include at least a device 104 that may be configured to generate a current or electrical signal, such as a signal capable of stimulating a signal or response from a target stimulation. For example, the device 104 may generate a current or electrical signal capable of stimulating an ECAP response from one or more nerves or an LFP response. In some examples, the device 104 may be referred to as a pulse generator. The pulse generator may be implantable in a patient or may be external to the patient. Additionally, the system 100 may include one or more wires or leads 108 that provide a connection between the device 104 and nerves of the patient for enabling the stimulation/blocking therapy.

Neuromodulation techniques (e.g., technologies that act directly upon nerves of a patient, such as the alteration, or "modulation," of nerve activity by delivering electrical impulses or pharmaceutical agents directly to a target area) may be used for assisting in treatments for different diseases, disorders, or ailments of a patient, such as chronic pain. Accordingly, as described herein, the neuromodulation techniques may be used for blocking pain signals sent to a patient's brain to relieve chronic pain. For example, the device 104 may provide electrical stimulation to one or more nerves in the spinal cord of the patient (e.g., via the one or more leads 108) to block the pain signals from being received by the brain. In other examples, the device 104 may provide electrical stimulation to the brain.

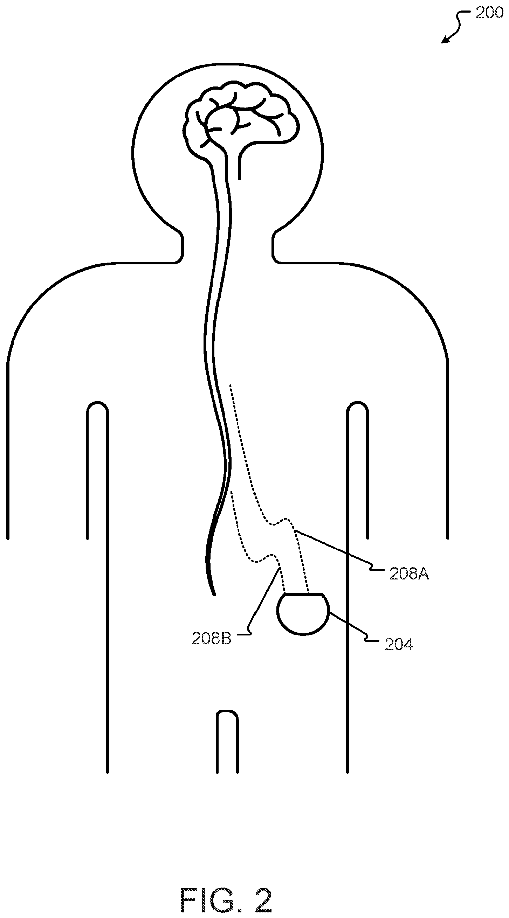

In some examples, as shown in Fig. 1, the one or more leads 108 may include one lead 108. In other embodiments, as will be described in Fig. 2, the one or more leads 108 may include multiple leads 208A, 208B. The lead 108 may be implanted on or near any target anatomical element. In some examples, the lead 108 is implanted near the spinal cord and more specifically, in the epidural space between the spinal cord and the vertebrae. Once implanted, the lead 108 may provide an electrical signal (whether stimulating or blocking) from the device 104 to the target anatomical element (e.g., one or more nerves in the spinal cord, the brain, etc.). The device 104 in some embodiments, may be implanted in the patient, though in other embodiments - such as during testing of the lead 108 - the device 104 may be external to the patient's body.

In some examples, the lead 108 may provide the electrical signals to the respective nerves via electrodes that are connected to the nerves (e.g., sutured in place, wrapped around the nerves, etc.). In some examples, the lead 108 may be referenced as cuff electrodes or may otherwise include the cuff electrodes (e.g., at an end of the lead 108 not connected or plugged into the device 104). Additionally or alternatively, while shown as physical wires that provide the connection between the device 104 and the one or more nerves, the electrodes may provide the electrical signals to the one or more nerves wirelessly (e.g., with or without the device 104).

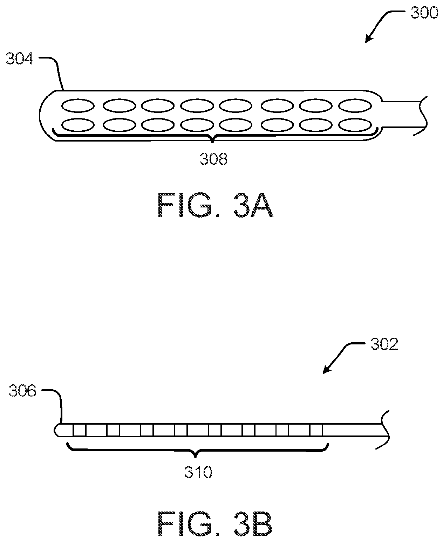

The electrodes may comprise stimulating electrodes (e.g., electrodes configured to stimulate a target anatomical element) or recording electrodes (e.g., electrodes configured to record a physiological response to the stimulation). In some embodiments, such as illustrated in Fig. 1, the lead 108 may comprise both stimulating electrodes and recording electrodes. In other embodiments, as described in Fig. 2, one lead may comprise stimulating electrodes and another lead may comprise recording electrodes. Example leads 108 and electrode configurations will be described in detail in Figs. 3A-3F, 4A-4C, and 5A-5C.

The stimulating electrodes may stimulate a target anatomical element such as a nerve and the recording electrodes may record a physiological response to the stimulation. More specifically in closed loop SCS, the recording electrode may record or measure the ECAP, which may be used to regulate or adjust the electrical signal generated by the device 104. For example, as a patient bends over, a distance between the lead 108 and the spinal cord (or any target anatomical element) may change, thus the resulting stimulation may be weaker or stronger based on the change in the distance. The recording electrode may measure and record the ECAPs and a processor may determine a difference in the ECAP. The difference may be used to adjust the electrical signal to cause an amplitude of the ECAP to remain within a therapeutic range that is comfortable for the patient. In other embodiments, the ECAP may be used to modulate therapy provided based on the ECAP signal whether or not there is movement of the patient, contacts, anatomical elements, stimulation target, etc. In other closed-loop neuromodulation therapies, other signals may be recorded. For example, in deep brain stimulation, local field potentials (LFPs) may be recorded in the brain.

In some embodiments, the stimulating electrodes and the recording electrodes may be synced via telemetry so as to coordinate a recording by the recording electrodes when the stimulating electrodes generate a stimulation. In other words, the recording electrode can be, for example, timed to begin recording at a time period or within a time period of the stimulating electrode generating a stimulation. In other embodiments where they system 100 includes a plurality of stimulating electrodes, one stimulating electrode may be generating a stimulation and another stimulating electrode may be multiplexed such that a timing of the stimulation of the one stimulating electrode does not interfere with a timing of a recording of a stimulation of the other stimulating electrode.

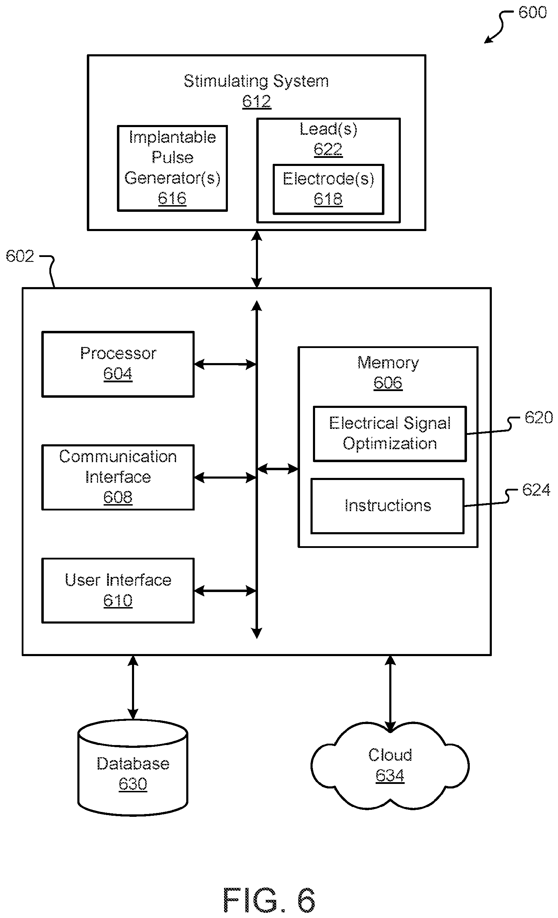

Additionally, while not shown, the system 100 may include one or more processors (e.g., one or more DSPs, general purpose microprocessors, graphics processing units, ASICs, FPGAs, or other equivalent integrated or discrete logic circuitry) that are programmed to carry out one or more aspects of the present disclosure. In some examples, the one or more processors may be used to drive a feedback loop in a closed-loop spinal cord stimulation system, as will be discussed in detail with respect to Figs. 6-7. In other examples, the one or more processors may include a memory or may be otherwise configured to perform the aspects of the present disclosure. For example, the one or more processors may provide instructions to the device 104, the electrodes, or other components of the system 100 not explicitly shown or described with reference to Fig. 1 for providing the pain blocking therapy to regulate chronic pain in a patient as described herein. In some examples, the one or more processors may be part of the device 104 or part of a control unit for the system 100 (e.g., where the control unit is in communication with the device 104 and/or other components of the system 100).

The system 100 or similar systems may be used, for example, to carry out one or more aspects of any of the method 700 described below. The system 100 or similar systems may also be used for other purposes. It will be appreciated that the human body has many nerves and the stimulation or blocking therapies described herein may be applied to any one or more nerves, which may reside at any location of a patient (e.g., lumbar, thoracic, extremity, brain, trunk etc.). The stimulation or blocking therapies may also be applied to any stimulation target (e.g., an anatomical element, non-neuronal cells, etc.)

Fig. 2 depicts a system 200 according to at least one embodiment of the present disclosure is shown. The system 200 is the same as or similar to the system 100 and comprises a device 204 which may be the same as or similar to the device 104. The system 200 also includes a lead that comprises a first lead 208A and a second lead 208B. The first lead 208A may comprise stimulating electrodes configured to stimulate a target anatomical element (e.g., a nerve) and the second lead 208B may comprise recording electrodes configured to record a physiological response to the stimulation. The first lead 208A and the second lead 208B may be implanted near each other in in the same space (for example, the epidural space), or may be implanted in separate spaces. In some embodiments, the first lead 208B may be oriented to, for example, an anatomical element such as a spinal cord or any neural element and the second lead 208B may be offset 90 degrees (e.g., perpendicular) to the first lead 208A. In other embodiments where the electrodes of the second lead 208B comprise recording electrodes, the recording electrodes may face different directions. For example, at least one recording electrode may be oriented towards the anatomical element such as the spinal cord or any neural element and another recording electoral may be oriented away from the anatomical element.

It will be appreciated that in some embodiments, the leads 108, 208A, 208B may comprise one lead, two leads, or more than two leads. In still other embodiments, the system 200 may not include a lead. In such embodiments, the device 104 (e.g., a pulse generator) may provide the stimulation. In still other embodiments, the device 104 may provide stimulation through one or more electrodes.

Fig. 3A and Fig. 3B depict a schematic illustration of a first lead 300 and a second lead 302, respectively. The first lead 300, as illustrated, comprises a paddle lead 304 and the second lead 302 comprises a cylindrical lead 306. It will be appreciated that while a paddle lead and a cylindrical lead are shown and described, any type of lead may be used to carry out any aspect of the present disclosure.

The paddle lead 304 may enable directional stimulation such that stimulation can be directed in a target direction. For example, the paddle lead 304 may be implanted above an anatomical element such as the spinal cord so that electrodes 308 on the paddle lead 304 face the spinal cord. During stimulation, the electrodes 308 direct the stimulation in the direction of the spinal cord.

The cylindrical lead 306 may also provide directional stimulation when the cylindrical lead 306 is segmented, as described in detail below. Further, the cylindrical lead 306 may be beneficially implanted using a minimally invasive surgical procedure (as opposed to forming an incision to implant the lead). During such procedures, the cylindrical lead 306 can be inserted into the epidural space using an epidural needle.

In the illustrated embodiments, the paddle lead 304 comprises sixteen electrodes 308 and the cylindrical lead 306 comprises eight electrodes 310. It will be appreciated that in other embodiments, the paddle lead 304 may comprise less than or more than sixteen electrodes and the cylindrical lead 306 may comprise less than or more than eight electrodes. Though the electrodes 308 of the paddle lead 304 are shown as ovals, the electrodes 308 (and/or the electrodes 310 of the cylindrical lead 306) may be any shape or size and may be spaced from each other at any distance. Each electrode may also be a different shape or size than another electrode and each electrode may be spaced a different distance from adjacent electrodes.

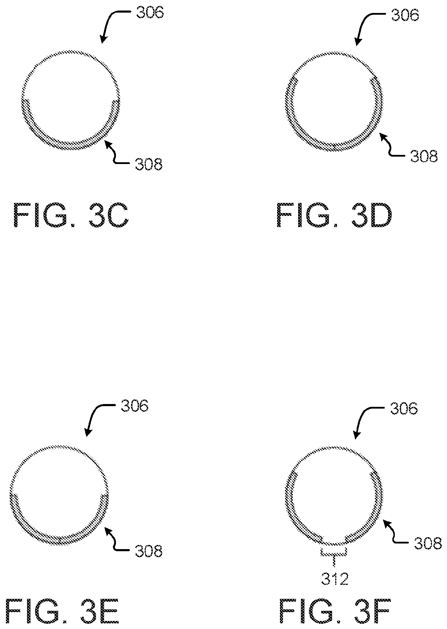

Further, though the electrodes 310 of the cylindrical lead are shown as ring electrodes, the cylindrical lead 306 may be segmented such that the electrodes 310 do not wrap around the entire cylindrical lead 306 as shown in the schematic illustration of cross-sectional views of the lead 306 of Figs. 3C-3F. More specifically, the cylindrical lead 306 can be segmented into any number of segments. For example, the cylindrical lead 306 can be bi-segmented or tri-segmented. In a segmented cylindrical lead 306, the electrode can be positioned in a segment such that the electrode will direct the stimulation in the direction that the electrode is facing. In other words, a segmented cylindrical lead 306 may enable directional stimulation in a target direction. In such designs, to accommodate recording electrodes, two recording electrodes may make up each half of a segmented contact. In other embodiments, the at least one recording contact or electrode 308 may comprise a single directional contact (shown in, for example, Fig. 3C), one or more directional 1/3 circumference contacts (shown in, for example, Fig. 3D), and/or one or more directional 1/4 circumference contacts (shown in, for example, Fig. 3E). In embodiments where the at least one recording contact or electrode comprise two or more contacts, the contacts may be spaced apart an axial inter-contact distance 312 (shown in, for example, Fig. 3F).

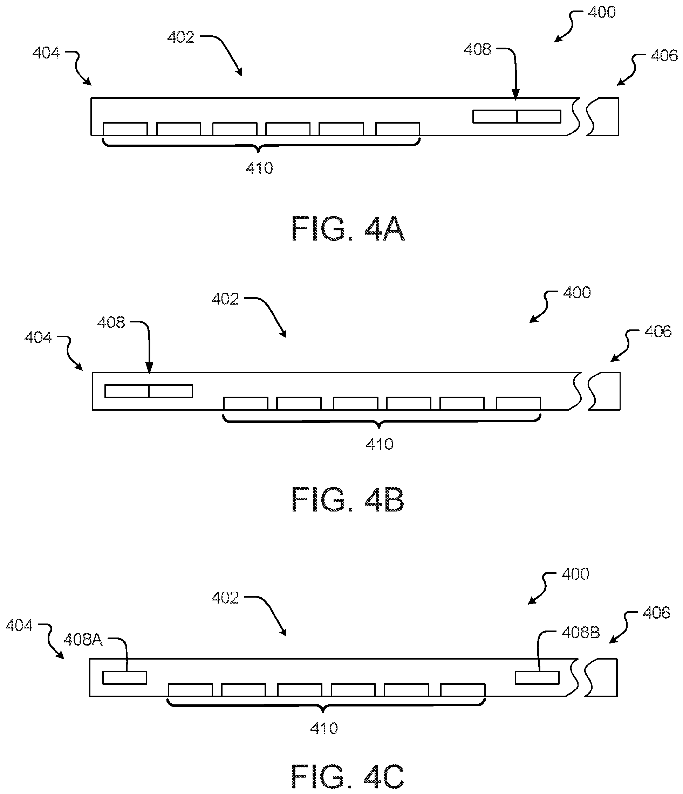

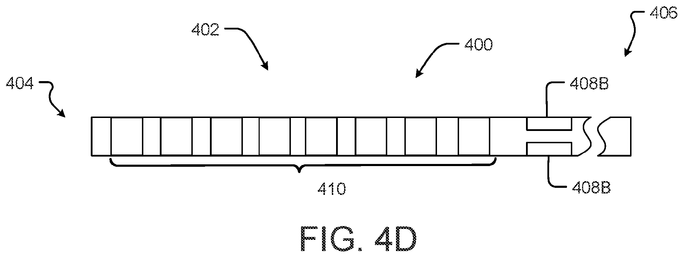

Figs. 4A, 4B, 4C, and 4D depict a schematic illustration of a lead 400 having a plurality of electrodes 402 in a first configuration, the lead 400 having the plurality of electrodes 402 in a second configuration, the lead 400 having the plurality of electrodes 402 in a third configuration, and the lead 400 having the plurality of electrodes 402 in a fourth configuration, respectively. Generally, a plurality of electrodes 402 is in communication with the lead 400. The plurality of electrodes 402 comprise at least one pair of stimulating electrodes 410 configured to stimulate a target anatomical element (based on an electrical signal generated by a device such as the device 104, 204) and at least one pair of recording electrodes 408 configured to record a physiological response resulting from the stimulation. In the illustrated embodiments, the at least one pair of stimulating electrodes 410 comprises three pairs of stimulating electrodes and the at least one pair of recording electrodes 408 comprises one pair of recording electrodes perpendicular to the at least one pair of stimulating electrodes 410. In other instances, the at least one pair of stimulating electrodes 410 may comprise one pair, two pairs, or more than two pairs of stimulating electrodes and the at least one pair of recording electrodes 408 may comprise one pair, two pairs, or more than two pairs of recording electrodes.

As shown, the at least one pair of recording electrodes 408 may be in different configurations relative to the at least one pair of stimulating electrodes 410. For example, in Fig. 4A, the at least one pair of recording electrodes 408 are at a distal end 406 to the at least one pair of stimulating electrodes 410; in Fig. 4B, the at least one pair of recording electrodes 408 are at a proximal end 404 to the at least one pair of stimulating electrodes 410; and in Fig. 4C, a first recording electrode 408A is at the proximal end 404 to the at least one pair of stimulating electrodes 410 and a second recording electrode 408B is at the distal end 406 to the at least one pair of stimulating electrodes 410.

As shown in Fig. 4D the at least one pair of stimulating electrodes 410 are oriented towards, for example, an anatomical element such as a spinal cord or any neural element and at least one of the at least one pair of recording electrodes 408 may be oriented towards the anatomical element and another one of the at least one pair of recording electrodes 408 may be oriented away from the anatomical element. In such examples, the at least one pair of stimulating electrodes 410 may be cylindrical and the at least one pair of recording electrodes 408 may be square and/or rectangular. In will be appreciated that in other examples, the at least one pair of stimulating electrodes 410 may be square and/or rectangular and/or the at least one pair of recording electrodes 410 may be cylindrical. Further, the at least one pair of recording electrodes 408 may be offset from the at least one pair of stimulating electrodes 410 and/or from each other.

Each of the plurality of electrodes 402 may be a different size and/or shape, though two or more of the plurality of electrodes 402 may have the same size and/or shape in some embodiments. Each of the plurality of electrodes 402 may also have a different surface area. For example, an electrode further away from a target anatomical element may have a surface area greater than an electrode closer to the target anatomical element. In another example, recording electrodes may have a surface area greater than or less than stimulating electrodes. Further, in some embodiments, the recording electrodes may be monopolar.

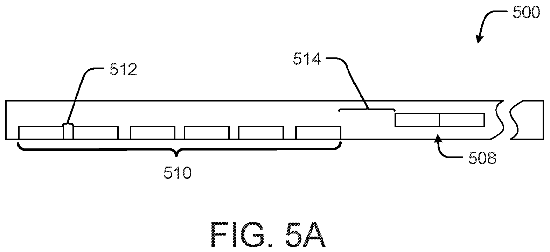

Figs. 5A-5C depict a schematic illustration of a lead 500 similar to or the same as the leads 108, 208A, 208B, 300, 302, 400. As shown, each stimulating electrode of at least one pair of stimulating electrodes 510 may be spaced apart at a first distance 512. The first distance 512 between each stimulating electrode may be the same as or different from each other. In other words, a distance between a first pair of stimulating electrodes may be different than a distance between a second pair of stimulating electrodes. The first distance 512 may be based on optimizing the spacing for, for example, SCS, though the spacing may be optimized for any type of stimulation therapy.

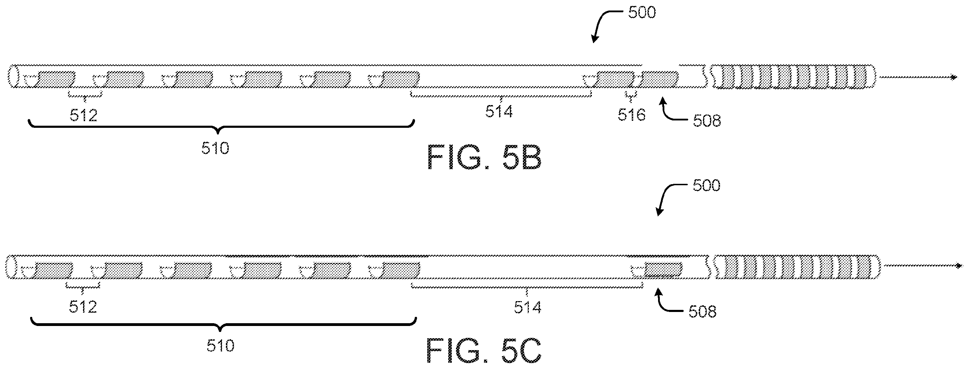

Also shown, the at least one pair of recording electrodes 508 are spaced a second distance 514 apart from the at least one pair of stimulating electrodes 510 in Figs. 5A and 5C. It will be appreciated that in some embodiments, the lead 500 may comprise one recording electrode 508, as shown in Fig. 5C, or may comprise more than two recording electrodes 508. The second distance 514 may be optimized to reduce stimulation artifacts in the data recorded by the at least one pair of recording electrodes 508. The second distance 514 may also be based on a conduction velocity of the neuron. The second distance 514 may be between about 30mm to about 60mm. In an exemplary embodiment, the second distance 514 may be about 56mm. It will be appreciated that in other embodiments, the second distance 514 may be less than 30mm or greater than 60mm. In still other embodiments, the second distance 514 may be optimized for SCS, peripheral nerve stimulation, sacral nerve stimulation, deep brain stimulation, or any other type of stimulation or therapy. Though not shown in Fig. 5, in some configurations, a first recording electrode may be spaced the second distance apart from the at least one pair of stimulating electrodes 510 on a first side of the at least one pair of stimulating electrodes 510 and a second recording electrode may be spaced a third distance apart from the at least one pair of stimulating electrodes 510 on a second side of the at least one pair of stimulating electrodes 510. The second distance and the third distance may be different distances. In other instances, the second distance and the third distance may be the same.

To further reduce stimulation artifacts, the at least one pair of recording electrodes 508 may be offset from the at least one pair of stimulating electrodes 510. In some embodiments, the at least one pair of recording electrodes 508 are offset 90 degrees from the at least one pair of stimulating electrodes 510. In other embodiments, the at least one pair of recording electrodes 508 are offset 270 degrees from the at least one pair of stimulating electrodes 510. In embodiments where the at least one pair of recording electrodes 508 and the at least one pair of stimulating electrodes 510 are on a single lead, the at least one pair of recording electrodes 508 may be offset from the at least one pair of stimulating electrodes 510 on the lead. In embodiments where the at least one pair of recording electrodes 508 is on a separate lead from the at least one pair of stimulating electrodes 510 (as described in, for example, Fig. 2), the lead having the at least one pair of recording electrodes 508 may be positioned at an offset from the lead having the at least one pair of stimulating electrodes 510.

Fig. 6 depicts a block diagram of a system 600 according to at least one embodiment of the present disclosure is shown. In some examples, the system 600 may implement aspects of or may be implemented by aspects of Figs. 1-5 as described herein. For example, the system 600 may be used with a pulse generator 616 (which may be implanted or external to a patient) and/or one or more lead(s) 622, and/or carry out one or more other aspects of one or more of the methods disclosed herein. The pulse generator 616 may represent an example of the device 104, 204 or a component of the device 104, 204 as described with reference to Figs. 1 and 2. The lead(s) 622 may represent an example of the lead(s) 108, 208A, 208B, 300, 302, 400, 500 or a component of the lead(s) 108, 208A, 208B, 300, 302, 400, 500 as described with reference to Figs. 1-5. The system 600 comprises a computing device 602, a stimulating/blocking system 612, a database 630, and/or a cloud or other network 634. Systems according to other embodiments of the present disclosure may comprise more or fewer components than the system 600. For example, the system 600 may not include one or more components of the computing device 602, the database 630, and/or the cloud 634.

The stimulating/blocking system 612 may comprise the pulse generator 616 and the lead 622. As previously described, the pulse generator 616 may be configured to generate a current, and the lead 622 may comprise a plurality of electrodes 618 configured to apply the current to a target anatomical element. The stimulating/blocking system 612 may communicate with the computing device 602 to receive instructions such as instructions 624 for applying an electrical signal to the target anatomical element, where the electrical signal is intended to block pain signals from being received by a patient's brain. The stimulating/blocking system 612 may also provide data (such as data received from recording electrodes such as recording electrodes 408, 508 capable of recording data), which may be used to optimize parameters of the electrical signal generated by the pulse generator 616, and/or to adjust parameters of the electrical signal to maintain an ECAP signal within a therapeutic range.

The computing device 602 is illustrated to include a processor 604, a memory 606, a communication interface 608, and a user interface 610. Computing devices according to other embodiments of the present disclosure may comprise more or fewer components than the computing device 602.

The processor 604 of the computing device 602 may be any processor described herein or any similar processor. The processor 604 may be configured to execute instructions 624 stored in the memory 606, which instructions may cause the processor 604 to carry out one or more computing steps utilizing or based on data received from the stimulating/blocking system 612, the database 630, and/or the cloud 634.

The memory 606 may be or comprise RAM, DRAM, SDRAM, other solid-state memory, any memory described herein, or any other tangible, non-transitory memory for storing computer-readable data and/or instructions. The memory 606 may store information or data useful for completing, for example, any steps of the method 700 described herein, or of any other methods. The memory 606 may store, for example, instructions and/or machine learning models that support one or more functions of the stimulating/blocking system 612. For instance, the memory 606 may store content (e.g., instructions 624 and/or machine learning models) that, when executed by the processor 604, cause the lead 622 to apply an electrical signal to a respective target anatomical element such as a nerve to block or regulate chronic pain.

The memory 606 may also store an electrical signal optimization 620. The electrical signal optimization 220 may correspond to a routine executed by the processor 604 to optimize the electrical signal used in an electrical stimulation and/or nerve blocking. Optimization may be achieved by adjusting signal frequency, adjusting signal type (e.g., square wave, sinusoidal wave, triangle wave, etc.), adjusting duty cycle, adjusting treatment duration, etc. More specifically, the electrical signal optimization 620 may enable the processor 604 to determine one or more parameters of the electrical signal. The electrical signal optimization 620 may also enable the processor 604 to determine or adjust one or more parameters of the electrical signal based on a physiological response recorded during stimulation of the target anatomical element. The one or more parameters may be adjusted to, for example, maintain an ECAP signal within a patient's therapeutic range.

Content stored in the memory 606, if provided as in instruction, may, in some embodiments, be organized into one or more applications, modules, packages, layers, or engines. Alternatively or additionally, the memory 606 may store other types of content or data (e.g., machine learning models, artificial neural networks, deep neural networks, etc.) that can be processed by the processor 604 to carry out the various method and features described herein. Thus, although various contents of memory 606 may be described as instructions, it should be appreciated that functionality described herein can be achieved through use of instructions, algorithms, and/or machine learning models. The data, algorithms, and/or instructions may cause the processor 604 to manipulate data stored in the memory 606 and/or received from or via the stimulating/blocking system 612, the database 630, and/or the cloud 634.

The computing device 602 may also comprise a communication interface 608. The communication interface 608 may be used for receiving data (for example, data from a recording electrodes capable of recording data) or other information from an external source (such as the stimulating/blocking system 612, the database 630, the cloud 634, and/or any other system or component not part of the system 600), and/or for transmitting instructions, images, or other information to an external system or device (e.g., another computing device 602, the stimulating/blocking system 612, the database 630, the cloud 634, and/or any other system or component not part of the system 600). The communication interface 608 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 608 may be useful for enabling the device 602 to communicate with one or more other processors 604 or computing devices 602, whether to reduce the time needed to accomplish a computing-intensive task or for any other reason.

The computing device 602 may also comprise one or more user interfaces 610. The user interface 610 may be or comprise a keyboard, mouse, trackball, monitor, television, screen, touchscreen, and/or any other device for receiving information from a user and/or for providing information to a user. The user interface 610 may be used, for example, to receive a user selection or other user input regarding any step of any method described herein. In some embodiments, the user interface 610 may be used to select one or more parameters for the electrodes including, but not limited to, selecting whether an electrode is active or inactive. For example, the user interface 610 may receive input to select a first electrode as active and to select a second and a third electrode as inactive. Notwithstanding the foregoing, any required input for any step of any method described herein may be generated automatically by the system 600 (e.g., by the processor 604 or another component of the system 600) or received by the system 600 from a source external to the system 600. In some embodiments, the user interface 610 may be useful to allow a surgeon or other user to modify instructions to be executed by the processor 604 according to one or more embodiments of the present disclosure, and/or to modify or adjust a setting of other information displayed on the user interface 610 or corresponding thereto.

Although the user interface 610 is shown as part of the computing device 602, in some embodiments, the computing device 602 may utilize a user interface 610 that is housed separately from one or more remaining components of the computing device 602. In some embodiments, the user interface 610 may be located proximate one or more other components of the computing device 602, while in other embodiments, the user interface 610 may be located remotely from one or more other components of the computer device 602.

Though not shown, the system 600 may include a controller, though in some embodiments the system 600 may not include the controller. The controller may be an electronic, a mechanical, or an electro-mechanical controller. The controller may comprise or may be any processor described herein. The controller may comprise a memory storing instructions for executing any of the functions or methods described herein as being carried out by the controller. In some embodiments, the controller may be configured to simply convert signals received from the computing device 602 (e.g., via a communication interface 608) into commands for operating the stimulating/blocking system 612 (and more specifically, for actuating the pulse generator 616 and/or electrodes 618 of the lead 622). In other embodiments, the controller may be configured to process and/or convert signals received from the stimulating/blocking system 612. Further, the controller may receive signals from one or more sources (e.g., the stimulating/blocking system 612) and may output signals to one or more sources.

The database 630 may store information such as patient data, results of a stimulation and/or blocking procedure, stimulation and/or blocking parameters, electrical signal parameters, electrode parameters, etc. The database 630 may be configured to provide any such information to the computing device 602 or to any other device of the system 600 or external to the system 600, whether directly or via the cloud 634. In some embodiments, the database 630 may be or comprise part of a hospital image storage system, such as a picture archiving and communication system (PACS), a health information system (HIS), and/or another system for collecting, storing, managing, and/or transmitting electronic medical records.

The cloud 634 may be or represent the Internet or any other wide area network. The computing device 602 may be connected to the cloud 634 via the communication interface 608, using a wired connection, a wireless connection, or both. In some embodiments, the computing device 602 may communicate with the database 630 and/or an external device (e.g., a computing device) via the cloud 634.

The system 600 or similar systems may be used, for example, to carry out one or more aspects of any of the method 700 as described herein. The system 600 or similar systems may also be used for other purposes.

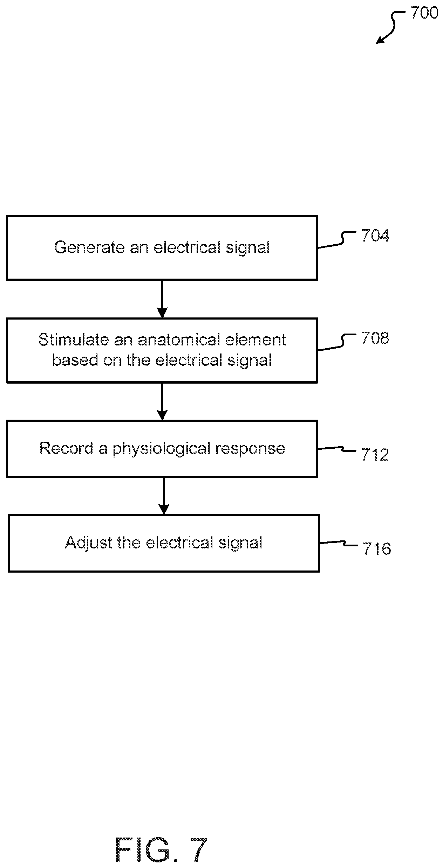

Fig. 7 depicts a method 700 that may be used, for example, to perform neuromodulation techniques (e.g., a stimulation/block therapy) to block or regulate pain signals.

The method 700 (and/or one or more steps thereof) may be carried out or otherwise performed, for example, by at least one processor. The at least one processor may be the same as or similar to the processor 604 or the processor(s) of the device 104 or 204 described above. The at least one processor may be part of the device 104 or 204 (such as a pulse generator) or part of a control unit in communication with the device 104 or 204. A processor other than any processor described herein may also be used to execute the method 700. The at least one processor may perform the method 700 by executing elements stored in a memory (such as a memory in the device 104 as described above or a control unit). The elements stored in the memory and executed by the processor may cause the processor to execute one or more steps of a function as shown in method 700. One or more portions of a method 700 may be performed by the processor executing any of the contents of memory, such as providing a stimulation/block therapy, executing an electrical signal optimization such as the electrical signal optimization 620, and/or any associated operations as described herein.

The method 700 comprises generating an electrical signal (step 704). The electrical signal may be generated by a device such as the device 104, 204 or a pulse generator such as the pulse generator 616. The device or the pulse generator may be implanted into a patient or may be stored external to the patient. The device or the pulse generator may operate automatically (using, for example, a processor, memory, controller, etc.) or may receive input from an external device such as, for example, a user device.

The method 700 also comprises stimulating an anatomical element based on the electrical signal (step 708). The anatomical element may be stimulated by at least one pair of stimulating electrodes such as the at least one pair of stimulating electrodes 410, 510. The at least one pair of stimulating electrodes may be in communication with the pulse generator via one or more leads such as the lead 108, 208A, 208B, 300, 302, 400, 500, 622 and may be configured to stimulate the anatomical element based on the electrical signal generated by the pulse generator in, for example, the step 704 above. In some embodiments the at least one pair of stimulating electrodes may be implanted near a spine and more specifically, may be implanted in the epidural space. In such embodiments, the anatomical element may be, for example, one or more nerves of the spinal cord. The anatomical element may be stimulated continuously in some embodiments or may be intermittently stimulated in other embodiments. In some applications such as spinal cord stimulation (SCS), one or more nerves may be stimulated to block pain signal(s) from being received by a patient's brain to regulate or reduce pain experienced by the patient.

The method 700 also comprises recording a physiological response (step 712). The physiological response may be recorded by at least one pair of recording electrodes such as the at least one pair of recording electrodes 408, 508. The at least one pair of recording electrodes may be on the same lead as the at least one pair of stimulating electrodes. In other instances, the at least one pair of recording electrodes may be on a separate lead from the at least one pair of stimulating electrodes. The at least one pair of recording electrodes may be proximal to the at least one pair of stimulating electrodes, distal to the at least one pair of stimulating electrodes, or one of the recording electrodes may be proximal to the at least one pair of stimulating electrodes and another one of the recording electrodes may be distal to the at least one pair of stimulating electrodes. To reduce stimulation artifacts that may interfere with recording and/or measuring the physiological response, the at least one pair of recording electrodes may be offset from the at least one pair of stimulating electrodes. In some embodiments, the at least one pair of recording electrodes are offset 90 degrees or 270 degrees from the at least one pair of stimulating electrodes. To also reduce stimulation artifacts, the at least one pair of recording electrodes may be spaced a distance from the at least one pair of stimulating electrodes. The distance may be, for example, between about 30mm and 60mm. In an exemplary embodiment, the distance is about 56mm.

The physiological response may be, for example, an ECAP that is measured in response to the stimulation executed in step 708 above. In such embodiments, the physiological response may indicate movement of the at least one pair of stimulating electrodes relative to the target anatomical element. For example, as a patient moves (whether by bending over, walking, or otherwise) the lead and the plurality of stimulating electrodes may move further away from or closer to the target anatomical element (e.g., one or more nerves) and thus the resulting stimulation may be stronger or weaker. For SCS, it is desirable to maintain an amplitude of the ECAP within a therapeutic range. When the patient moves, the resulting stimulation may result in the ECAP moving out of the therapeutic range. Thus, it is desirable to adjust the electrical signal in real-time in response to the movement of the lead relative to the target anatomical element. In other embodiments, the physiological response may be, for example, LFPs or any other signal resulting from a neuromodulation therapy.

The method 700 also comprises adjusting one or more parameters of the electrical signal (step 716). The one or more parameters of the electrical signal may be adjusted by a processor such as the processor 604 or a processor of the pulse generator. The step 716 may occur when the physiological response recorded in the step 712 either meets or exceeds a predetermined threshold or when the physiological response is outside of a predetermined range. In some embodiments, the processor may receive the recorded physiological response from the step 712 and may input the recorded physiological response to an electrical signal optimization such as the electrical signal optimization 620. The processor may also execute the electrical signal optimization to determine one or more adjusted parameters of the electrical signal.

In some embodiments, the steps 712 and 716 may be repeated and the electrical signal may be continuously adjusted to maintain the physiological response within a target range or threshold. In some embodiments, the physiological response measured and recorded may be the ECAPs (or LFPs, or any other signal) resulting from the stimulation. In such embodiments, the steps 712 and 716 may be repeated to maintain the amplitude of the ECAP within the therapeutic range. For example, the electrical signal may be adjusted in the step 716, the ECAP may be recorded in the step 712, and the electrical signal may be adjusted again in the step 716 if the ECAP is not within the therapeutic range.

It will be appreciated that the steps 704, 708, 712, and/or 716 may be repeated continuously separately or simultaneously. For example, the steps 704, 708, and 712 may be ongoing and the step 712 may not occur until a change in the physiological response is detected.

The present disclosure encompasses embodiments of the method 700 that comprise more or fewer steps than those described above, and/or one or more steps that are different than the steps described above.

As noted above, the present disclosure encompasses methods with fewer than all of the steps identified in Fig. 7 (and the corresponding description of the method 700), as well as methods that include additional steps beyond those identified in Fig. 7 (and the corresponding description of the method 700). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, as defined by the appended claims.

## Claims

1. A system for controlling a therapeutic procedure comprising:
a pulse generator (616) configured to generate an electrical signal;
a lead (622) in communication with the pulse generator (616) and configured to transmit the electrical signal to a plurality of electrodes (618); and
the plurality of electrodes (618) in communication with the lead (622) and configured to be implanted near a stimulation target, the plurality of electrodes (618) comprising at least one stimulating electrode and at least one recording electrode, the at least one stimulating electrode configured to stimulate an anatomical element based on the electrical signal and the at least one recording electrode configured to record a physiological response,
wherein the at least one stimulating electrode comprises a pair of stimulating electrodes;
wherein the at least one recording electrodes comprises a pair of recording electrodes;
wherein the at least one pair of recording electrodes is arranged in a substantially perpendicular configuration to a configuration of the at least one pair of stimulating electrodes.

2. The system of claim **1,** wherein the at least one recording electrode is proximal to the at least one stimulating electrode.

3. The system of claim **1,** wherein the at least one recording electrode is distal to the at least one stimulating electrode.

4. The system of any of the preceding claims, wherein a first recording electrode of the at least one recording electrode is proximal to the at least one stimulating electrode and a second recording electrode of the at least one recording electrode is distal to the at least one stimulating electrode.

5. The system of any of the preceding claims, wherein the lead (622) comprises a directional lead and wherein the at least one stimulating electrode directs the stimulation in a target direction.

6. The system of any of the claims 1 to 4, wherein the lead comprises a directional lead and wherein the at least one recording electrode records the physiological response in a target direction.

7. The system of any of the preceding claims, wherein each stimulating electrode of the pair of stimulation electrodes is spaced apart from one another at a first distance and the at least one pair of stimulating electrodes are positioned at a second distance from the at least one recording electrode.

8. The system of any of the preceding claims, wherein the lead comprises a cylindrical lead having segments and the at least one recording electrode comprises at least a half segment.

9. The system of any of the preceding claims, wherein the at least one recording electrode has a surface area larger than the at least one stimulating electrode.

10. The system of any of the preceding claims, further comprising:
a processor; and
a memory storing data for processing by the processor, the data, when processed, causes the processor to:
generate an electrical signal using the pulse generator;
stimulate the anatomical element based on the electrical signal;
record the physiological response from the stimulation; and
adjust the electrical signal based on the recorded physiological response.

## Patentansprüche

1. System zur Steuerung eines therapeutischen Verfahrens, umfassend:
einen Impulsgenerator (616), der konfiguriert ist, um ein elektrisches Signal zu erzeugen;
einen Lead (622) in Kommunikation mit dem Impulsgeber (616) und konfiguriert, um das elektrische Signal zu einer Vielzahl von Elektroden (618) zu übertragen; und
die Vielzahl von Elektroden (618) in Verbindung mit dem Lead (622) und konfiguriert, um nahe einem Stimulationsziel implantiert zu werden, wobei die Vielzahl von Elektroden (618) mindestens eine Stimulationselektrode und mindestens eine Aufzeichnungselektrode umfasst, wobei die mindestens eine Stimulationselektrode konfiguriert ist, um ein anatomisches Element basierend auf dem elektrischen Signal zu stimulieren, und die mindestens eine Aufzeichnungselektrode konfiguriert ist, um eine physiologische Reaktion aufzuzeichnen,
wobei die mindestens eine Stimulationselektrode ein Paar von stimulierenden Elektroden umfasst;
wobei die mindestens eine Aufzeichnungselektrode ein Paar von Aufzeichnungselektroden umfasst;
wobei das mindestens eine Paar von Aufzeichnungselektroden in einer im Wesentlichen senkrechten Konfiguration zu einer Konfiguration des mindestens einen Paars von Stimulationselektroden angeordnet ist.

2. System nach Anspruch 1, wobei die mindestens eine Aufzeichnungselektrode proximal zu der mindestens einen Stimulationselektrode angeordnet ist.

3. System nach Anspruch 1, wobei die mindestens eine Aufzeichnungselektrode distal zu der mindestens einen Stimulationselektrode angeordnet ist.

4. System nach einem der vorstehenden Ansprüche, wobei eine erste Aufzeichnungselektrode der mindestens einen Aufzeichnungselektrode proximal zu der mindestens einen Stimulationselektrode angeordnet ist und eine zweite Aufzeichnungselektrode der mindestens einen Aufzeichnungselektrode distal zu der mindestens einen Stimulationselektrode angeordnet ist.

5. System nach einem der vorstehenden Ansprüche, wobei der Lead (622) einen gerichteten Lead umfasst und wobei die mindestens eine Stimulationselektrode die Stimulation in eine Zielrichtung richtet.

6. System nach einem der Ansprüche 1 bis 4, wobei der Lead einen gerichteten Lead umfasst und wobei die mindestens eine Aufzeichnungselektrode die physiologische Reaktion in einer Zielrichtung aufzeichnet.

7. System nach einem der vorstehenden Ansprüche, wobei jede Stimulationselektrode des Paars von Stimulationselektroden von der anderen in einem ersten Abstand beabstandet ist und das mindestens eine Paar von Stimulationselektroden in einem zweiten Abstand von der mindestens einen Aufzeichnungselektrode positioniert ist.

8. System nach einem der vorstehenden Ansprüche, wobei der Lead ein zylindrisches Lead mit Segmenten umfasst und die mindestens eine Aufzeichnungselektrode mindestens ein halbes Segment umfasst.

9. System nach einem der vorstehenden Ansprüche, wobei die mindestens eine Aufzeichnungselektrode einen größeren Oberflächenbereich als die mindestens eine Stimulationselektrode aufweist.

10. System nach einem der vorstehenden Ansprüche, ferner umfassend:
einen Prozessor; und
einen Speicher, der Daten zum Verarbeiten durch den Prozessor speichert, wobei die Daten, wenn sie verarbeitet werden, den Prozessor veranlassen zum:
Erzeugen eines elektrischen Signals unter Verwendung des Impulsgebers;
Stimulieren des anatomischen Elements basierend auf dem elektrischen Signal;
Aufzeichnen der physiologischen Reaktion auf die Stimulation; und
Anpassen des elektrischen Signals basierend auf die aufgezeichnete physiologische Reaktion.

## Revendications

1. Système permettant de commander une procédure thérapeutique comprenant :
un générateur d'impulsions (616) configuré pour générer un signal électrique ;
une sonde (622) en communication avec le générateur d'impulsions (616) et configurée pour transmettre le signal électrique à une pluralité d'électrodes (618) ; et
la pluralité d'électrodes (618) en communication avec la sonde (622) et configurées pour être implantées près d'une cible de stimulation, la pluralité d'électrodes (618) comprenant au moins une électrode de stimulation et au moins une électrode d'enregistrement, l'au moins une électrode de stimulation étant configurée pour stimuler un élément anatomique sur la base du signal électrique et l'au moins une électrode d'enregistrement étant configurée pour enregistrer une réponse physiologique,
dans lequel l'au moins une électrode de stimulation comprend une paire d'électrodes de stimulation ;
dans lequel l'au moins une électrode d'enregistrement comprend une paire d'électrodes d'enregistrement ;
dans lequel l'au moins une paire d'électrodes d'enregistrement est disposée dans une configuration sensiblement perpendiculaire à une configuration de l'au moins une paire d'électrodes de stimulation.

2. Système selon la revendication 1, dans lequel l'au moins une électrode d'enregistrement est proximale à l'au moins une électrode de stimulation.

3. Système selon la revendication 1, dans lequel l'au moins une électrode d'enregistrement est distale à l'au moins une électrode de stimulation.

4. Système selon l'une quelconque des revendications précédentes, dans lequel une première électrode d'enregistrement de l'au moins une électrode d'enregistrement est proximale à l'au moins une électrode de stimulation et une seconde électrode d'enregistrement de l'au moins une électrode d'enregistrement est distale à l'au moins une électrode de stimulation.

5. Système selon l'une quelconque des revendications précédentes, dans lequel la sonde (622) comprend une sonde directionnelle et dans lequel l'au moins une électrode de stimulation dirige la stimulation dans une direction cible.

6. Système selon l'une quelconque des revendications 1 à 4, dans lequel la sonde comprend une sonde directionnelle et dans lequel l'au moins une électrode d'enregistrement enregistre la réponse physiologique dans une direction cible.

7. Système selon l'une quelconque des revendications précédentes, dans lequel chaque électrode de stimulation de la paire d'électrodes de stimulation est espacée l'une de l'autre à une première distance et l'au moins une paire d'électrodes de stimulation est positionnée à une seconde distance de l'au moins une électrode d'enregistrement.

8. Système selon l'une quelconque des revendications précédentes, dans lequel la sonde comprend une sonde cylindrique ayant des segments et l'au moins une électrode d'enregistrement comprend au moins un demi-segment.

9. Système selon l'une quelconque des revendications précédentes, dans lequel l'au moins une électrode d'enregistrement présente une surface plus grande que l'au moins une électrode de stimulation.

10. Système selon l'une quelconque des revendications précédentes, comprenant en outre :
un processeur ; et
une mémoire stockant des données destinées à être traitées par le processeur, les données, lorsqu'elles sont traitées, amènent le processeur à :
générer un signal électrique à l'aide du générateur d'impulsions ;
stimuler l'élément anatomique sur la base du signal électrique ;
enregistrer la réponse physiologique de la stimulation ; et
ajuster le signal électrique sur la base de la réponse physiologique enregistrée.
